# EUROPÄISCHE PATENTSCHRIFT

(11) **EP 2 066 236 B1**
(45) Veröffentlichungstag und Bekanntmachung des Hinweises auf die Patenterteilung: **16.09.2015**
(21) Anmeldenummer: 07801261.4
(22) Anmeldetag: 16.08.2007
(51) Int. Cl.: A61B 5/087, G01N 33/497, A61B 5/097

(54) **VORRICHTUNG ZUM FRAKTIONIEREN DES EXSPIRATIONSVOLUMENS**
DEVICE FOR FRACTIONATING EXPIRATION VOLUME
DISPOSITIF DE FRACTIONNEMENT D'UN VOLUME D'EXPIRATION

(30) Priorität: 16.08.2006 DE 202006012837 U; 16.08.2006 DE 102006039140
(43) Veröffentlichungstag der Anmeldung: 10.06.2009
(73) Patentinhaber: Aerocrine AB, 169 67 Solna (SE)
(72) Erfinder: BECHER, Gunther, 16321 Bernau OT Schönow (DE); EICHLER, Rüdiger, 97225 Zellingen (DE)
(74) Vertreter: Holmberg, Martin Tor
(86) Internationale Anmeldenummer: PCT/DE2007/001471
(87) Internationale Veröffentlichungsnummer: WO 2008/019680

(56) Entgegenhaltungen:
- WO-A-03/073935
- WO-A-2005/079669
- DE-A1- 10 158 288
- US-A- 5 787 885

## Beschreibung

Die Erfindung betrifft eine Vorrichtung zum Fraktionieren des Exspirationsvolumen des Respirationstraktes, die einen Einlass für inspiratorische Luft in einen Hohlraum hinein und vom Hohlraum einen Auslass für inspiratorische Luft zur Lunge aufweist und im Einlass für inspiratorische Luft ein Mittel zum Verschließen des Einlasses eingerichtet ist. Der Hohlraum ist mit einem Einlass für exspiratorische Luft und mindestens einem Auslass für die exspiratorische Luft ausgestattet. Im Auslass für die exspiratorische Luft ist ein Mittel zum Verschließen des Auslass eingerichtet. Zur Messung des Volumenstroms ist im Einlass für inspiratorische Luft oder im Hohlraum ein Durchflussmesser eingerichtet, der mit einer Steuereinheit verbunden ist. Aus dem gemessenen Wert des Volumenstroms der inspiratorischen Luft wird mittels der Steuereinheit ein korrelierender theoretischer Wert eines Volumenstroms der exspiratorischen Luft errechnet und/oder es ist in der Steuereinheit ein korrelierender theoretischer Wert eines Volumenstroms der exspiratorischen Luft gespeichert und von dort abrufbar. Mittels der Steuereinheit ist der theoretische Wert des Volumenstrom der exspiratorischen Luft in theoretische Werte von Teilvolumenströme des Volumenstromes zerlegbar. Den Teilvolumenströmen sind unterschiedlichen Zonen des Respirationstraktes zuordenbar.

Das Mittel zum Verschließen des Einlass für die inspiratorische Luft und/oder das Mittel zum Verschließen des Auslasses oder der Auslässe für die exspiratorischen Luft sind durch die Steuereinheit ansteuerbar und regelbar, wobei der Betriebszustand "zu" oder "auf" des Mittels zum Verschließen Einlass für inspiratorische Luft und/oder des Mittels zum Verschließen des Auslasses oder der Auslässe für exspiratorische Luft entsprechend einem bestimmten Teilvolumenstrom des Volumenstroms der exspiratorischen Luft einstellbar ist. Außerdem betrifft die Erfindung ein Verfahren zur Fraktionierung sowie eine Verwendung einer solchen Vorrichtung.

Solche Vorrichtungen werden beispielsweise zur Untersuchung der Funktion der Lunge sowie zur Diagnose von krankhaften oder altersbedingten Veränderungen eingesetzt. Insbesondere wird das Exspirationsvolumen nach Markern, die nicht dem eigentlichen Atemgas entstammen, untersucht, die ein Hinweis oder Nachweis krankhafter Veränderungen sind. Derartige krankhafte Veränderungen können diverse Lungenkrebsformen, Bronchitis, infektiöse chronische obstruktive Lungenerkrankungen, Bronchiektasen, Lungenemphysem und Pneumonien sein. Auch können bakterielle oder Pilz- bzw. Hefeinfektionen nachgewiesen werden. Die Aufzählung ist nicht abschließend. Solche Marker können flüchtige oder auch nicht flüchtige anorganische oder organische Verbindungen sein.

Durch die WO 91/05255 A1 ist ein Verfahren zur Diagnose des Gesundheitszustandes sowie zur Therapieüberwachung und zur Verlaufskontrolle von Krankheiten, speziell der Lunge und der Atemwege, bekannt, bei dem nicht-flüchtige Substanzen - gegebenenfalls zusammen mit endogenen und exogen flüchtigen Stoffen - in der menschlichen Atemluft analysiert werden. Hierzu werden von einem Probanden Atemstöße auf ein auf -196°C gekühltes, 1cm² großes Probenträgerplättchen aufgehaucht. Die Probe wird dann auf dem Cryotisch durch Anlagen eines Ultrahochvakuums gefriergetrocknet und anschließend in unterschiedlicher Weise analysiert.

Aus der EP 0 759 169 B1 ist ein Verfahren zum Sammeln von ausgeatmetem Atemkondensat für die Diagnose des Gesundheitszustandes und von Stoffwechselleistungen der Lunge und der Atemwege sowie anderer Organe und zur Analyse ausgeatmeter körperfremder Stoffe, bekannt, bei dem die Ausatemluft ein Probensammelrohr durchströmt und in diesem auf eine Minustemperatur unter 0°C abgekühlt wird, wobei die flüssigen und lösbaren Bestandteile auskondensieren und an der, eine niedrige Temperatur als das Atemkondensat aufweisenden, Innenwand des Probensammelrohres anfrieren.

Die DE 199 51 204 C2 beschreibt ein Verfahren zur Analyse der Inhaltstoffe der Ausatemluft, bei dem aus der Ausatemluft gewonnenes Atemkondensat einer Analyse unterzogen und das Ergebnis angezeigt wird, wobei die Menge des aus der Ausatemluft gewonnenen Atemkondensates gemessen wird und nach dem Erreichen einer vorgegebenen Probenmenge eine direkte Bestimmung der enthaltenen Substanzen durch Messung von Einzel- und/oder Summenparametern mittels eines oder mehreren elektrochemischen Sensoren erfolgt.

Ein weiteres Verfahren zur Bestimmung von Parametern eines Atemkondensats unter Verwendung eines oder mehrer Sensoren zum Messen der Parameter des Atemkondensats und einer Auswerteeinheit mit Anzeige für die Messergebnisse, wird in der DE 101 37 565 B4 beschrieben.

US 5,787,885 betrifft eine Vorrichtung und ein Verfahren zur Probennahme und Prüfung des Atems von Menschen auf Erkrankungen in der Speiseröhre, des Magens oder des Gastrointestinaltraktes durch die automatische Analyse der chemischen Inhalte der abgetasteten detektierten Luft umfassend einen Behälter und ein mikroprozessorgesteuertes Ventil zur Auswahl eines Teils des Atems um es in den Behälter nach dem Ausatmen durch einen Einlaß in den Behälter einzufügen und zu speichern, um so das Speichern der ersten Luft ausgeatmeten Luft zu vermieden. Ein normalerweise offener Druckschalter oder Sensor der durch den Druck des anfänglichen Flusses des Atems in ein Einlaßrohr von der Mündung aktiviert wird, liefert ein Startsignal zum Steuern eines elektronischen Zeitgebers oder eines Mikroprozessors, der danach den Abtastvorgang steuert. Die Vorrichtung enthält auch Durchgänge, mehrere Sammelkammern, um mehrere Proben aus den gleichen oder verschiedenen Körperflüssigkeiten zu sammeln und mit einem in sich geschlossenen Sensor oder einem Fernanalysesystem zu analysieren.

DE 10158288 präsentiert ein Verfahren zur Entnahme von Gas- bzw. Geruchsproben aus der Ausatemluft, welches zur Bestimmung einer bakteriellen Besiedlung oder zur Identifikation von Stoffwechselvorgängen bei der Diagnose der Atemwege dient. Interne Volumenanteile des Exhalates werden in einem externen Totraumvolumen abgebildet und sind damit hersichtlich ihrer Herkunft definierbar und einer speziellen gassensorischen Untersuchung zuführbar. Gemäß einer erfindungsgemäßen Anordnung zur Durchführung des Verfahrens ist als externes Totraumvolumen ein Gefäß mit einem Volumen vorgesehen, das etwa dem des Ausatemvolumens entspricht, dem die Ausatemluft über ein Mundstück und ein Ventil zugeführt wird und an dem ein Absaugestutzen angeordnet ist, über den die Volumenanteile des Exhalates einem Gassensor zuführbar sind.

Nachteilig an denen im Stand der Technik genannten Verfahren und Vorrichtungen zur Analyse des Exspirationsvolumens und zur Bestimmung von Markern pulmonaler Erkrankungen ist, dass das gesamte Exspirationsvolumen auf Marker untersucht werden muss, um feststellen zu können, dass eine Erkrankung vorliegt. Nachdem ein Marker gefunden wurde, bleibt der untersuchende Arzt jedoch darüber im Unklaren, an welchem Ort im Respirationstrakts die Erkrankung lokalisiert ist. Hier müssen in der Regel weitere Untersuchungen, wie Ultraschall, Röntgen, Computertomographie etc. folgen. Im umgekehrten Fall, es sei eine Erkrankung der Lunge durch Röntgen oder andere Untersuchungsmethoden entdeckt worden, muss wiederum das gesamte Exspirationsvolumen analysiert werden, um die die entdeckte Veränderung oder Erkrankung spezifizierenden Markern zu detektieren und über diese eine exakte Diagnose zu stellen.

Dies bedingt mindestens zwei aufeinander folgende Untersuchungsschritte, die einen hohen zeitlichen und finanziellen Aufwand bedeuten.

Aus dem Stand der Technik sind des Weiteren Vorrichtungen bekannt, mit welchen nur ein Teilstrom des Exspirationsvolumens analysiert werden muss. Hierbei handelt es sich um Bypass-Vorrichtungen, bei welchen ein Teilexspirationsvolumenstrom, der vom Hauptexspirationsvolumenstrom abgezweigt und parallel zu diesem über einen Detektionssensor oder in ein Analysegerät geführt wird. Hierdurch wird das zu analysierende Exspirationsvolumen verkleinert. Diese bautechnische Abwandlung ändert nichts an der Notwendigkeit, den Ort der Erkrankung oder Veränderung mittels einer weiteren Untersuchung bestimmen zu müssen.

Das der Erfindung zu Grunde liegende Problem ist es daher, eine Vorrichtung anzugeben, mit welchem mittels einer einzigen Untersuchung des Exspirationsvolumens Ort einer pulmonalen Veränderung detektiert werden kann.

Die vorliegende Erfindung löst dieses technische Problem, indem es eine Vorrichtung zum Fraktionieren des Exspirationsvolumens angibt, die im Anspruch 1 definiert ist und die einen Einlass für inspiratorische Luft in einen Hohlraum hinein und vom Hohlraum einen Auslass für inspiratorische Luft zur Lunge aufweist und im Einlass für inspiratorische Luft ein Mittel zum Verschließen des Einlasses eingerichtet ist. Der Hohlraum ist mit einem Einlass für exspiratorische Luft und mindestens einem Auslass für die exspiratorische Luft ausgestattet. Im Auslass für die exspiratorische Luft ist ein Mittel zum Verschließen des Auslass eingerichtet. Zur Messung des Volumenstroms ist im Einlass für inspiratorische Luft oder im Hohlraum ein Durchflussmesser eingerichtet, der mit einer Steuereinheit verbunden ist. Aus dem gemessenen Wert des Volumenstroms der inspiratorischen Luft wird mittels der Steuereinheit ein korrelierender theoretischer Wert eines Volumenstroms der exspiratorischen Luft errechnet und/oder es ist in der Steuereinheit ein korrelierender theoretischer Wert eines Volumenstroms der exspiratorischen Luft gespeichert und von dort abrufbar. Mittels der Steuereinheit ist der theoretische Wert des Volumenstrom der exspiratorischen Luft in theoretische Werte von Teilvolumenströme des Volumenstromes zerlegbar. Den Teilvolumenströmen sind unterschiedlichen Zonen des Respirationstraktes zuordenbar.

Die Vorrichtung wird direkt an den Mund des Patienten gesetzt oder ist an eine Leitung, die zum Mund des Patienten führt, angeschlossen. Der Patient atmet durch die Vorrichtung hindurch ein- und aus, vorzugsweise nicht gegen eine Stenose.

Eine in- oder exspiratorische regelbare Stenose (Strömungswiderstand) ist insofern vorgesehen , dass der Patient durch Atmung gegen diese unwillkürlich zu einer bestimmten gewünschten Atmung animiert wird. Weiter ist eine exspiratorische Stenose (exspiratorischer Widerstand) in der Lage, den Atemfluss und die Weite der Atemwege dahingehend zu manipulieren, dass der Gasaustausch und die Abatmung von zur Analyse vorgesehenen Stoffen standardisiert wird. Damit werden wiederholbare Atemmanöver möglich, zu denen der Proband /Patient unwillkürlich veranlasst wird.

Sind in der Anmeldung nur Mittel zum Verschließen genannt, so sind sowohl die Mittel zum Verschließen des Einlass sowie des Auslass oder der Auslässe gemeint, als auch Mittel zu einem teilweisen Verschluss bzw. zur teilweisen Freigabe des Atemstromes um eine bestimmten Strömungswiderstand zu generieren. Im Idealfall wird der Verschluss und teilweise Verschluss über eine regelbare Schließvorrichtung realisiert, vorzugsweise sind dies Ventile, mit denen der Querschnitt des Auslasses auf jede beliebige Weite verengt bzw. erweitert werden kann.

Das Mittel zum Verschließen des Einlass für die inspiratorische Luft und/oder das Mittel zum Verschließen des Auslasses oder der Auslässe für die exspiratorischen Luft sind durch die Steuereinheit ansteuerbar und regelbar, wobei der Betriebszustand "zu" oder "auf" des Mittels zum Verschließen Einlass für inspiratorische Luft und/oder des Mittels zum Verschließen des Auslasses oder der Auslässe für exspiratorische Luft entsprechend einem bestimmten Teilvolumenstrom des Volumenstroms der exspiratorischen Luft einstellbar ist. Außerdem gibt die Erfindung ein Verfahren zur Fraktionierung sowie eine Verwendung einer solchen Vorrichtung zur Lösung des technischen Problems an.

Mit der Vorrichtung können zeitlich nacheinander folgende Teilvolumenströme eines Exspirationsvolumens separiert werden, wobei diesen Teilabschnitten bestimmte Regionen und Zonen des Respirationstraktes zugewiesen werden können. Dies ist darin begründet, dass die Strömung in der Lunge durch die leitenden Atemwege überwiegend laminar und lediglich an Verzweigungsstellen und Einengungen der Bronchien turbulent ist. Daraus folgt, dass sich Gase, die unterschiedlichen Lungenzonen entstammen, beim Ausatmen nicht oder nur wenig vermischen.

Die Lunge ist grob in drei Hauptzonen, der Leitungszone, der Übergangszone und der Respirationszone unterteilt. Jede Hauptzone ist wiederum in weitere Unterzonen aufgegliedert. Der Anteil der einzelnen Zonen an der Gesamtgröße der Lunge sind als Durchschnittswerte für Menschen aller Altersgruppe und Geschlechts Fachliteratur entnehmbar.

Prinzipiell ist das Exspirationsvolumen etwas kleiner als das Inspirationsvolumen, weil weniger CO₂ abgegeben als O₂ aufgenommen wird. Jedoch kann durch die Erwärmung des Atemgases in der Lunge die Proportionen in die Richtung verschoben werden, dass das erwärmte Exspirationsvolumen größer als das Inspirationsvolumen ist.

Das exspiratorische Atemzugvolumen setzt sich aus 2 Volumenanteilen zusammen, aus dem ausgeatmeten Volumen des Todraums und dem Alveolarraum. Beim Ausatmen wird zuerst das Gas aus dem anatomischen Todraum, wie Nase bzw. Mund, Pharynx, Larynx, Trachea, Bronchien und Bronchiolen entfernt. Das Volumen des Todraums hängt wiederum von der Körpergröße und der Körperposition des Patienten ab. Anschließend werden die Gasvolumenanteile des Alveolarraums respiriert.

Bisher erfolgte eine getrennte Erfassung beider Volumenanteile nur über indirekte Messverfahren.

Die Erfindung stellt über die Messung des inspiratorischen Luftvolumenstroms auf Basis der prozentualen Volumenanteile der einzelnen Lungenzonen am Gesamtlungenvolumen und unter der Berücksichtigung, dass das Exspirationsvolumen etwas kleiner als das Inspirationsvolumen ist, eine Korrelation zu der Größe einzelner, zeitlich aufeinaderfolgender Teilvolumenströme des exspiratorischen Luftvolumenstroms her.

Beim Einatmen (Inspiration, Inhalation) wird der aktuelle Volumenstrom gemessen. Diesem Wert wird ein theoretischer Wert eines fiktiven Exspirationsvolumenstromes zugeordnet, welcher auf Kenntnis des Quotienten von Exspirationsvolumen zu Inspirationsvolumen in einer Steuereinheit wie beispielsweise einem Rechner errechnet werden kann oder von einem in der Steuereinheit gespeicherten Speicherdatensatz abgerufen werden kann. Die Korrektur der Differenz von Inhalations- zu Exhalationsvolumen aufgrund physiologischer Gegebenheiten (unterschiedliche Gasanteile im Inhalat und Exhalat, Temperaturen des Inhalats und des Exhalts, etc.) kann mittels einer programmgestützten BTPS-Korrektur erfolgen.

Gleichzeitig wird das fiktive Exspirationsvolumen mittels der Steuereinheit in theoretische Teilvolumenströmen unterteilt. Die Größe und Anzahl der zu separierenden Teilvolumenströme kann über ein Bedienpaneel in die Steuereinheit eingegeben oder aus in der Steuereinheit gespeicherten Datensätzen abgerufen werden. Nach Größe und Anzahl der theoretischen Teilvolumenströme erfolgt die Fraktionierung des realen Exspirationsvolumen in entsprechende reale Teilvolumenströme.

Setzt nun die Ausatmung (Exspiration, Exhalation) ein, kann dieses Ereignis durch einen Widerstandsmesser oder anderen Messeinrichtungen, wie beispielsweise Strömungsmesser erfasst und der Steuereinheit ein Signal gesendet werden. Auch kann das Ende der Einatmung als Signal verwendet werden. Dieses Signal kann mittels der Messwerterfassung am Durchflussmessers generiert werden.

Die Mittel zum Verschließen sind mit der Steuereinheit verbunden und können mittels der Steuereinheit angesteuert werden. Vorzugsweise ist immer nur ein Mittel zum Verschließen im Betriebszustand "auf", während die übrigen im Betriebszustand "zu" sind. Der Zeitpunkt und die Dauer des Betriebszustandes "auf" bzw. "zu" eines jeden Mittels zum Verschließen erfolgt nach Maßgabe der Größe des zu separierenden Teilvolumenstroms. Die einzelnen Mittel zum Verschließen werden zeitlich nacheinander einzeln geöffnet und geschlossen.

Jeder separierte Teilvolumenstrom entspricht dann bzw. entstammt dann einer definierten Zone des Respirationstraktes. Vorteilhaft an der Erfindung ist, dass jeder Atemzug des Patienten einzeln erfasst und selektiv fraktioniert wird. Hierdurch werden Messwertstreuungen minimiert und die Messgenauigkeit erhöht.

Die Fraktionen mehrerer Atemzüge d.h. mehrere Exspirationsvolumen können auch gesammelt und vereinigt werden, wobei es sich versteht, dass die Teilvolumenströme entstammend einer bestimmten Zone des Respirationstraktes immer im gleichen Mittel zum Speichern gesammelt, bzw. durch den gleichen Auslass geführt werden, d.h. es nicht zu einer Vermischung verschiedner Teilvolumenströme verschiedener Zonen kommt.

In einer besonderen Ausführungsform kann an einem oder an mehreren Auslässen für die exspiratorische Luft ein Mittel zum Speichern des Teilvolumenstromes angebracht sein. Das Mittel zum Speichern des Teilvolumenstroms ist vorteilhafterweise von der Vorrichtung abnehmbar und verschließbar, um die separierten Teilvolumenströme entweder für einer spätere Analyse oder einen weiteren Aufarbeitungsschritt aufzubewahren und/oder einem separaten Gerät zuführen zu können.

Es kann der separierte Teilvolumenstrom auch direkt in ein Gerät zur Analyse der Inhaltstoffe des Teilvolumenstroms eingeleitet werden, welches vorzugsweise direkt am Auslass für die exspiratorische Luft eingerichtet ist bzw. vom Auslass ein Leitung direkt in das Gerät führt, so dass eine online-Bestimmung der Inhaltsstoffe erfolgen kann.

Das Einleiten des Teilvolumenstroms in das Analysegerät kann auf Grundlage der durch das Ausatmen induzierten Strömung erfolgen. Alternativ kann der Teilvolumenstrom mit einer Pumpe, die im Auslass oder in der Zuleitung zum Analysegerät eingerichtet sein kann, in das Analysegerät befördert werden. Vorzugsweise ist das Gerät zur Analyse des Teilvolumenstroms aus der Gruppe bestehend aus "Ionenbeweglichkeitsspektrometer (IMS), Gaschromatograph, HPLC, Maldi-TOF, Massenspektrometer, Biosensoren, elektrochemische Sensoren" ausgewählt.

Die Mittel zum Verschließen sind aus der Gruppe bestehend aus "Pneumatisches Ventil, elektromagnetisches Ventil, Rückschlagventil" ausgewählt.

In einem Nebenanspruch lehrt die Erfindung des Weiteren ein Verfahren zum Fraktionieren des Exspirationsvolumens, das im Anspruch 8 definiert ist, aufweisend die Verfahrensschritte:
a) Messen des Volumenstroms der inspiratorischen Luft
b) Zuordnen des Messwertes nach Schritt a) einem theoretischen Wert eines zur Größe des inspiratorischen Volumenstroms korrelierenden exspiratorischen Volumenstroms, wobei der theoretische Wert eines exspiratorische Volumenstroms in theoretische Teilvolumenströme zerlegt ist und diese theoretischen Teilvolumenströme unterschiedlichen Zonen des Respirationstraktes zugeordnet sind
c) Zerlegen des tatsächlichen exspiratorischen Volumenstroms in Teilvolumenströme, wobei der reale Teilvolumenstrom nach Maßgabe der Größe des theoretischen Teilvolumenstroms isoliert und/oder verworfen wird.

Vorzugsweise wird der erste Teilvolumenstrom, einfachster weise über den geöffneten Einlass für die inspiratorische Luft, verworfen. Der erste Teilvolumenstrom enthält in der Regel nur das Exhalationsgas aus Mund- und Rachenraum, eventuell auch aus tieferen Bereichen des anatomischen Todraums, welche für die Untersuchung erkrankter Lungenzonen eher von untergeordnetem Interesse sind.

In einer bevorzugten Ausführungsform erfolgt das Zerlegen (Fraktionieren) des tatsächlichen exspiratorischen Volumenstroms in Teilvolumenströme nach durch die Länge der theoretischen Teilvolumenströme vorgegebenen Zeitintervallen. Der tatsächliche exspiratorische Volumenstrom kann aber auch nach Maßgabe der Größe des Teilvolumens des theoretischen Teilvolumenstroms in reale Teilvolumenströme fraktioniert werden.

Nach der Fraktionierung des Exspirationsvolumens, entsprechend den Verfahrensschritten a), b) und c) in Teilvolumenströme, kann mittels eines markerspezifischen Testsystems jeder Teilvolumenstrom auf Marker von Lungenkrankheiten untersucht werden. Bestimmte Marker stehen oder sind Hinweise für bestimmte Lungenerkrankungen. Solche Marker sind beispielsweise Wasserstoffperoxid als Marker der weißen Blutkörperchen (Neutrophile) Stickstoffmonoxid als Marker der Aktivität des Endothels und/oder der eosinophilen Blutzelle., aber auch Leukotriene als Marker der Entzündung, Zytokine als regulatorische Proteine, und auch Tumormarker.

Die abgetrennten Teilvolumenströme werden in einem weiteren Verfahrensschritt unterschiedlichen Zonen des Respirationstraktes zugewiesen. Die Zuweisung kann manuell oder auch rechnergestützt erfolgen. Durch diesen Verfahrensschritt wird eine Korrelation von detektierten Markern von Lungenerkrankungen und dem Ort der Lungenerkrankung hergestellt. Danach kann die Erkrankung oder auch mehrere Erkrankungen in einem oder aber auch in verschiedenen Zonen des Respirationstraktes lokalisiert und auf Basis der Informationen eine gezielte Therapie entwickelt werden.

Des Weiteren lehrt die Erfindung die Verwendung einer Vorrichtung zur Fraktionierung des Exspirationsvolumens zur Lokalisierung von Erkrankungen im Respirationstrakt, wobei die aus dem Exspirationsvolumen abgetrennten Teilvolumina unterschiedlichen Zonen des Respirationstraktes zugeordnet sind.

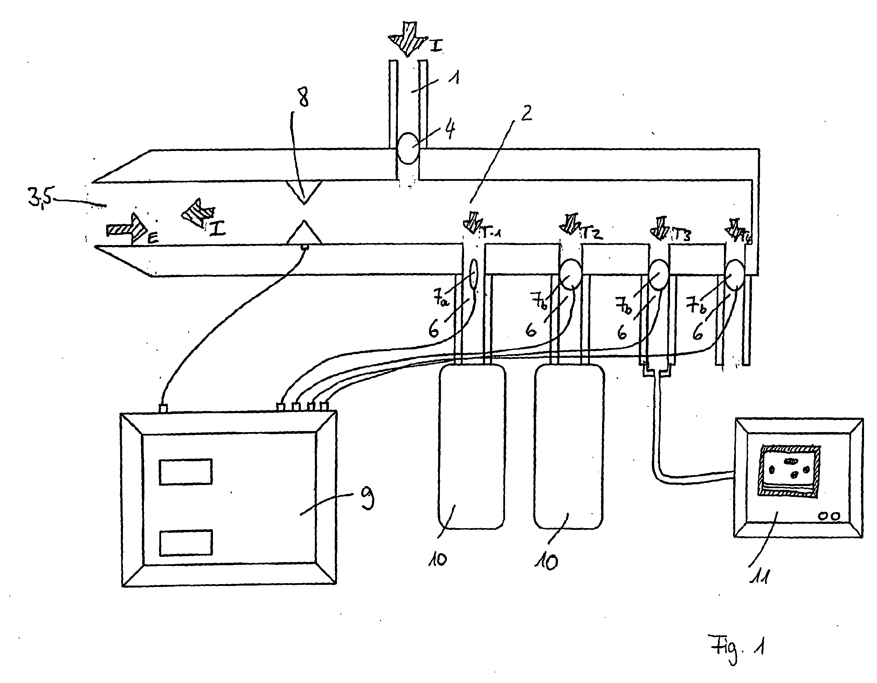

Nachstehend wird die Erfindung anhand eines Ausführungsbeispiels näher erläutert. Figur 1 zeigt eine Vorrichtung zum Fraktionieren des Exspirationsvolumens mit einem Einlass für inspiratorische Luft 1, einem Hohlraum 2 sowie einem Auslass für inspiratorische Luft 3, der in diesem Ausführungsbeispiel gleichzeitig der Einlass für exspiratorische Luft 5 ist. Diese beiden können aber auch getrennt sein. Diese Öffnung wird direkt an den Mund des Patienten gesetzt oder an eine Leitung, die zum Mund des Patienten führt, angeschlossen. Der Patient atmet durch die Vorrichtung hindurch ein- und aus. Im Einlass für inspiratorische Luft 1 ist ein Mittel zum Verschließen des Einlasses eingerichtet, welches sich im Betriebzustand zu befindet. Die Vorrichtung ist mit vier Auslässen für exspiratorische Luft 6 ausgestatten. In den Auslässen sind Mittel zum Verschließen des Auslasses eingerichtet, wobei das Mittel zum Verschließen des Auslasses 7a im Betriebszustand "auf" ist, die anderen Mittel zum Verschließen des Auslasses 7b im Betriebszustand "zu" sind. Im Hohlraum 2 ist ein Durchflussmesser 8 installiert, der zum Messen des eingehenden und auch ausgehenden Volumenstrom eingerichtet ist. Er kann aber auch nur eingerichtet sein, um den eingehenden Volumenstrom zu messen. Der Durchflussmesser 8 ist mit einer Steuereinheit 9 verbunden, an die die Messwerte übermittelt werden. Die Steuereinheit 9 ist zudem mit den Auslässen für exspiratorische Luft 7a, 7b verbunden. An zwei Auslässen für exspiratorische Luft sind Mittel zum Speichern des Teilvolumenstroms 11 angebracht. Vorzugsweise sind dies gasundurchlässige Behälter z.B. gasdichte Tüten. Ein Auslass für exspiratorische Luft (7b) ist über eine Leitung direkt an ein Gerät zur Analyse der Inhaltsstoffe des Teilvolumenstroms 11 angeschlossen, mittels welchem der über diesen Auslass separierte Teilvolumenstrom nach Markern für Lungenerkrankungen untersucht werden kann.

Die mit I gekennzeichneten Pfeile geben die Strömungsrichtung der inspiratorischen Luft, die mit E gekennzeichneten Pfeile die der exspiratorischen Luft an. Die Pfeile T1 bis T4 bezeichnen die Teilvolumenströme 1 bis 4, die den Zonen 1 bis 4 des Respirationstraktes zugeordnet werden können.

### Bezugszeichenliste.

- 1: Einlass für inspiratorische Luft
- 2: Hohlraum
- 3: Auslass für inspiratorische Luft
- 4: Mittel zum Verschließen des Einlasses
- 5: Einlass für exspiratorische Luft
- 6: Auslass für exspiratorische Luft
- 7: Mittel zum Verschließen des Ausslass
- 8: Durchflussmesser
- 9: Steuereineheit
- 10: Mittel zum Speichern des Teilvolumenstroms
- 11: Gerät zur Analyse der Inhaltsstoffe des Teilvolumenstroms
- a: Betriebszustand "auf"
- b: Betriebszustand "zu"

## Patentansprüche

1. Vorrichtung zum Fraktionieren des Exspirationsvolumens,
wobei die Vorrichtung einen Einlass für inspiratorische Luft (1) in einen Hohlraum (2) hinein und vom Hohlraum (2) einen Auslass für inspiratorische Luft (3) zur Lunge aufweist,
wobei im Einlass für inspiratorische Luft (1) ein Mittel zum Verschließen des Einlasses (4) eingerichtet ist,
wobei der Hohlraum (2) einen Einlass für exspiratorische Luft (5) aufweist,
wobei der Hohlraum (2) mindestens einen Auslass für die exspiratorische Luft (6) aufweist,
wobei in dem mindestens einen Auslass für die exspiratorische Luft (6) ein Mittel zum Verschließen des mindestens einen Auslasses (7a, 7b) eingerichtet ist,
wobei mindestens ein Durchflußmesser (8) zur Messung des Volumenstroms der inspiratorischen Luft im Einlass für inspiratorische Luft (1) oder im Hohlraum (2) eingerichtet ist und der Durchflußmesser (8) zur Messung des Volumenstroms der inspiratorischen Luft mit einer Steuereinheit (9) verbunden ist,
wobei mittels der Steuereinheit (9) aus dem gemessenen Wert des Volumenstroms der inspiratorischen Luft ein korrelierender theoretischer Wert eines Volumenstroms der exspiratorischen Luft errechenbar ist und/oder ein korrelierender theoretischer Wert eines Volumenstroms der exspiratorischen Luft in der Steuereinheit (9) speicherbar und abrufbar ist,
wobei mittels der Steuereinheit (9) der theoretische Wert des Volumenstrom der exspiratorischen Luft in theoretische Werte von Teilvolumenströme des Volumenstromes zerlegbar ist und die Teilvolumenströme unterschiedlichen Zonen des Respirationstraktes zuweisbar sind,
wobei das Mittel zum Verschließen des Einlasses (4) für die inspiratorische Luft und/oder das Mittel zum Verschließen des mindestens einen Auslasses oder der Auslasse (7a, 7b) für die exspiratorischen Luft durch die Steuereinheit (9) ansteuerbar und regelbar sind,
wobei der Betriebszustand "zu" (a) oder "auf" (b) des Mittels zum Verschließen des mindestens einen Auslasses für exspiratorische Luft (7a, 7b) entsprechend einem bestimmten Teilvolumenstrom des Volumenstroms der exspiratorischen Luft einstellbar ist, und die einzelnen Mittel zum Verschließen (4, 7) zeitlich nacheinander einzeln im Betriebszustand "auf" und Betriebszustand "zu" sein können, wobei die Zeitspanne und Dauer des Betriebszustand "auf" bzw. "zu" nach Maßgabe der Länge der theoretischen Teilvolumenströme mittels der Steuereinheit (9) einstellbar und regelbar ist.

2. Vorrichtung zum Fraktionieren des Exspirationsvolumens nach Anspruch 1, **dadurch gekennzeichnet, dass** am mindestens einen Auslass für die exspiratorische Luft (7a, 7b) ein Mittel zum Speichern des Teilvolumenstromes (10) angebracht ist.

3. Vorrichtung zum Fraktionieren des Exspirationsvolumens nach Anspruch 1 oder 2, **dadurch gekennzeichnet, dass**
der Einlass für inspiratorische Luft (4) im Betriebszustand "zu" ist, wenn mindestens ein Auslass für exspiratorische Luft (7a, 7b) im Betriebszustand "auf" ist.

4. Vorrichtung zum Fraktionieren des Exspirationsvolumens nach einem der Ansprüche 1 bis 3, **dadurch gekennzeichnet, dass**
ein Gerät zur Analyse der Inhaltstoffe (11) des Teilvolumenstroms, vorzugsweise direkt an einem Auslass für die exspiratorische Luft (7a, 7b), eingerichtet ist.

5. Vorrichtung zum Fraktionieren des Exspirationsvolumens nach Anspruch 4, **dadurch gekennzeichnet, dass**
das Gerät zur Analyse der Inhaltsstoffe des Teilvolumenstroms (11) ausgewählt ist aus der Gruppe bestehend aus "Ionenbeweglichkeitsspektrometer, Gaschromatograph, HPLC, Maldi-TOF, Massenspektrometer, Biosensoren, Elektrochemische Sensoren".

6. Vorrichtung zum Fraktionieren des Exspirationsvolumens nach Anspruch 2, **dadurch gekennzeichnet, dass** das Mittel zum Speichern des Teilvolumenstroms (10) verschließbar und von der Vorrichtung abnehmbar ist.

7. Vorrichtung zum Fraktionieren des Exspirationsvolumens nach einem der Ansprüche 1 bis 6, **dadurch gekennzeichnet, dass**
die Mittel zum Verschließen (4, 7) ausgewahlt sind aus der Gruppe bestehend aus "Pneumatisches Ventil, elektromagnetisches Ventil, Rückschlagventil".

8. Verfahren zum Fraktionieren des Exspirationsvolumens, aufweisend die Verfahrensschritte:
a) Messen des Volumenstroms der inspiratorischen Luft,
b) Zuordnen des Messwertes nach Schritt a) einem theoretischen Wert eines zur Größe des inspiratorischen Volumenstroms korrelierenden exspiratorischen Volumenstroms, wobei der theoretische Wert eines exspiratorische Volumenstroms in theoretische Teilvolumenströme zerlegt ist und diese theoretischen Teilvolumenströme unterschiedlichen Zonen des Respirationstraktes zugeordnet sind
c) Zerlegen des tatsächlichen exspiratorischen Volumenstroms in Teilvolumenströme, wobei der reale Teilvolumenstrom nach Maßgabe der Größe des theoretischen Teilvolumenstroms isoliert und/oder verworfen wird, und
das Zerlegen des tatsächlichen exspiratorischen Volumenstroms in Teilvolumenströme nach durch die Länge der theoretischen Teilvolumenströme vorgegebenen Zeitintervallen erfolgt, oder
das Zerlegen des tatsächlichen exspiratorischen Volumenstroms in Teilvolumenströme nach durch die Größe der theoretischen Teilvolumenströme vorgegebene Teilvolumen erfolgt

9. Verfahren zum Fraktionieren des Exspirationsvolumens nach Anspruch 8 **dadurch gekennzeichnet, dass**
der erste Teilvolumenstrom, vorzugsweise über den Einlass für die inspiratorische Luft (1, 4) im Betriebszustand 'auf' verworfen wird.

10. Verfahren zum Fraktionieren des Exspirationsvolumens nach einem der Ansprüche 8 oder 9, **dadurch gekennzeichnet, dass**
nach den Schritten a), b) und c) der Teilvolumenstrom mittels eines marker-spezifischen Testsystems auf Marker von Lungenkrankheiten untersucht wird.

11. Verfahren zum Fraktionieren des Exspirationsvolumens nach einem der Ansprüche 8 bis 10, **dadurch gekennzeichnet, dass**
die abgetrennten Teilvolumenströme unterschiedlichen Zonen des Respirationstraktes zugewiesen werden.

12. Verwendung einer Vorrichtung nach einem der Ansprüche 1 bis 7 zur Fraktionierung des Exspirationsvolumens zur Lokalisierung von Veränderungen im Respirationstrakt, wobei die aus dem Exspirationsvolumen abgetrennten Teilvolumina unterschiedlichen Zonen des Respirationstraktes zugeordnet sind.

## Claims

1. Apparatus for fractionating expiration volume,
wherein the apparatus comprises an inspiratory air inlet (1) into a cavity (2) and an inspiratory air outlet (3) from the cavity (2) to the lungs,
wherein a means (4) for closing the inlet is installed in the inspiratory air inlet (1), wherein the cavity (2) comprises an expiratory air inlet (5),
wherein the cavity (2) comprises at least one outlet (6) for the expiratory air, wherein a means (7a, 7b) for closing the at least one outlet is installed in the at least one outlet (6) for the expiratory air,
wherein at least one flowmeter (8) for measuring the volume flow of the inspiratory air is installed in the inspiratory air inlet (1) or in the cavity (2) and the flowmeter (8) for measuring the volume flow of the inspiratory air is connected to a control unit (9),
wherein a correlating theoretical value of a volume flow of the expiratory air can be calculated by the control unit (9) from the measured value of the volume flow of the inspiratory air and/or a correlating theoretical value of a volume flow of the expiratory air can be stored and called up in the control unit (9),
wherein the theoretical value of the volume flow of the expiratory air can be broken down by the control unit (9) into theoretical values for partial volume flows of the volume flow and the partial volume flows can be assigned to different zones of the respiratory tract,
wherein the means (4) for closing the inlet for the inspiratory air and/or the means (7a, 7b) for closing the at least one outlet or the outlets for the expiratory air can controlled and regulated by the control unit (9),
wherein the "closed" (a) or "open" (b) operating state of the means (7a, 7b) for closing the at least one expiratory air outlet can be set according to a specific partial volume flow of the volume flow of the expiratory air,
and the individual closing means (4, 7) can successively and individually be in the "open" operating state and the "closed "operating state, wherein the time period and duration of the "open" and "closed" operating states respectively can be set and regulated by the control unit (9) according to the length of the theoretical partial volume flows.

2. Apparatus for fractionating expiration volume according to claim 1, **characterised in that** a means (10) for storing the partial volume flow is attached to at least one outlet (7a, 7b) for the expiration air.

3. Apparatus for fractionating expiration volume according to either claim 1 or claim 2, **characterised in that** the inspiratory air inlet (4) is in the "closed" operating state when at least one expiratory air outlet (7a, 7b) is in the "open" operating state.

4. Apparatus for fractionating expiration volume according to any of claims 1 to 3, **characterised in that** a device (11) for analysing the contents of the partial volume flow, preferably directly at an outlet (7a, 7b) for the expiratory air, is installed.

5. Apparatus for fractionating expiration volume according to claim 4, **characterised in that** the device (11) for analysing the contents of the partial volume flow is selected from the group consisting of "ion mobility spectrometer, gas chromatograph, HPLC, MALDI-TOF, mass spectrometer, biosensors, electrochemical sensors".

6. Apparatus for fractionating expiration volume according to claim 2, **characterised in that** the means (10) for storing the partial volume flow can be closed and removed from the apparatus.

7. Apparatus for fractionating expiration volume according to any of claims 1 to 6, **characterised in that** the closing means (4, 7) are selected from the group consisting of "pneumatic valve, electromagnetic valve, non-return valve".

8. Method for fractionating expiration volume comprising the method steps of:
a) measuring the volume flow of the inspiratory air,
b) assigning the measurement value from step a) to a theoretical value of an expiratory volume flow corresponding to the size of the inspiratory volume flow, wherein the theoretical value of an expiratory volume flow is broken down into theoretical partial volume flows and said theoretical partial volume flows are assigned to different zones of the respiratory tract
c) breaking down the actual expiratory volume flow into partial volume flows, wherein the real partial volume flow is isolated and/or discarded according to the size of the theoretical partial volume flow, and
breaking down the actual expiratory volume flow into partial volume flows occurs according to time intervals predetermined by the length of the theoretical partial volume flows,
or
breaking down the actual expiratory volume flow into partial volume flows occurs according to partial volumes predetermined by the size of the theoretical partial volume flows.

9. Method for fractionating expiration volume according to claim 8, **characterised in that** the first partial volume flow is discarded, preferably via the inlet (1, 4) for the inspiratory air in the "open" operating state.

10. Method for fractionating expiration volume according to either claim 8 or claim 9, **characterised in that**, following steps a), b) and c), the partial volume flow is inspected for markers of lung diseases by means of a marker-specific test system.

11. Method for fractionating expiration volume according to any of claims 8 to 10, **characterised in that** the separated partial volume flows are allocated to different zones of the respiratory tract.

12. Use of a device according to any of claims 1 to 7 for fractionating expiration volume in order to localise changes in the respiratory tract, wherein the partial volumes separated from the expiration volume are assigned to different zones of the respiratory tract.

## Revendications

1. Dispositif de fractionnement du volume d'expiration,
dans lequel le dispositif présente une entrée (1) pour l'air d'inspiration dans un espace creux (2) et une sortie (3) pour l'air d'inspiration de l'espace creux (2) vers les poumons,
dans lequel est disposé à l'entrée (1) pour l'air d'inspiration un moyen (4) pour fermer l'entrée,
dans lequel l'espace creux (2) présente une entrée (5) pour l'air d'expiration,
dans lequel l'espace creux (2) présente au moins une sortie (6) pour l'air d'expiration,
dans lequel est disposé dans la au moins une sortie (6) pour l'air d'expiration un moyen (7a, 7b) pour fermer la au moins une sortie,
dans lequel au moins un débitmètre (8) pour mesurer le flux volumique de l'air d'inspiration est monté dans l'entrée (1) pour l'air d'inspiration ou dans l'espace creux (2) et le débitmètre (8) pour mesurer le flux volumique de l'air d'inspiration est relié à une unité de commande (9),
dans lequel, au moyen de l'unité de commande (9), on peut calculer à partir de la valeur mesurée du flux volumique de l'air d'inspiration une valeur théorique corrélée d'un flux volumique de l'air d'expiration et/ou on peut mémoriser et interroger une valeur théorique corrélée d'un flux volumique de l'air d'expiration dans l'unité de commande (9),
dans lequel, au moyen de l'unité de commande (9), la valeur théorique du flux volumique de l'air d'expiration peut être décomposée en valeurs théoriques de flux volumiques partiels du flux volumique et les flux volumiques partiels peuvent être affectés à différentes zones des voies respiratoires,
dans lequel le moyen (4) permettant de fermer l'entrée pour l'air d'inspiration et/ou le moyen (7a, 7b) pour fermer la au moins une sortie ou les sorties pour l'air d'expiration peut ou peuvent être commandés et régulés par l'unité de commande (9),
dans lequel l'état de fonctionnement « fermé » (a) ou « ouvert » (b) du moyen (7a, 7b) pour la fermeture de la au moins une sortie pour l'air d'expiration peut être réglé en fonction d'un flux volumique partiel déterminé du flux volumique de l'air d'expiration et les moyens individuels de fermeture (4, 7) peuvent être momentanément l'un après l'autre individuellement à l'état de fonctionnement « ouvert » et à l'état de fonctionnement « fermé », dans lequel l'intervalle de temps et la durée de l'état de fonctionnement « ouvert » ou « fermée » peuvent être réglés et régulés en fonction de la longueur des flux volumiques partiels théoriques au moyen de l'unité de commande (9).

2. Dispositif de fractionnement du volume d'expiration selon la revendication 1, **caractérisé en ce que** l'on dispose sur au moins une sortie (7a, 7b) pour l'air d'expiration un moyen (10) permettant de mémoriser le flux volumique partiel.

3. Dispositif de fractionnement du volume d'expiration selon la revendication 1 ou la revendication 2, **caractérisé en ce que** :
l'entrée (4) pour l'air d'inspiration se trouve à l'état de fonctionnement « fermé » si au moins une sortie (7a, 7b) pour l'air d'expiration se trouve à l'état de fonctionnement « ouvert ».

4. Dispositif de fractionnement du volume d'expiration selon l'une quelconque des revendications 1 à 3, **caractérisé en ce que** :
un appareil (11) permettant d'analyser les contenus du flux volumique partiel est monté de préférence directement sur une sortie (7a, 7b) pour l'air d'expiration.

5. Dispositif de fractionnement du volume d'expiration selon la revendication 4, **caractérisé en ce que** :
l'appareil (11) pour l'analyse des contenus du flux volumique partiel est choisi dans le groupe constitué des suivants : spectromètre à mobilité ionique, chromatographe en phase gazeuse, HPLC, Maldi-TOF, spectromètre de masse, biocapteurs et capteurs électrochimiques.

6. Dispositif de fractionnement du volume d'expiration selon la revendication 2, **caractérisé en ce que** le moyen (10) permettant de mémoriser le flux volumique partiel peut être fermé et retiré du dispositif.

7. Dispositif de fractionnement du volume d'expiration selon l'une quelconque des revendications 1 à 6, **caractérisé en ce que** :
les moyens de fermeture (4, 7) sont choisis dans le groupe constitué des suivants : soupape pneumatique, soupape électromagnétique, soupape de non-retour.

8. Procédé de fractionnement du volume d'expiration présentant les étapes consistant à :
a) mesurer le flux volumique de l'air d'inspiration,
b) affecter la valeur de mesure selon l'étape a) à une valeur théorique d'un flux volumique d'expiration corrélé à la grandeur du flux volumique d'inspiration, dans lequel la valeur théorique d'un flux volumique d'expiration est décomposé en flux volumiques partiels théoriques et ces flux volumiques partiels théoriques sont affectés à différentes zones des voies respiratoires,
c) décomposer le flux volumique d'expiration réel en flux volumiques partiels, dans lequel le flux volumique partiel réel est isolé en fonction de la grandeur du flux volumique partiel théorique et/ou mis en rebut, et
la décomposition du flux volumique d'expiration réel en flux volumiques partiels se fait selon des intervalles de temps préétablis par la longueur des flux volumiques partiels théoriques, ou
la décomposition du flux volumique d'expiration réel en flux volumiques partiels se faisant selon des volumes partiels préétablis par la grandeur des flux volumiques partiels théoriques.

9. Procédé de fractionnement du volume d'expiration selon la revendication 8, **caractérisé en ce que** :
le premier flux volumique partiel est mis au rebut de préférence à l'entrée (1, 4) pour l'air d'inspiration à l'état de fonctionnement « ouvert ».

10. Procédé de fractionnement du volume d'expiration selon l'une quelconque des revendications 8 ou 9, **caractérisé en ce que** :
après les étapes a), b) et c), le flux volumique partiel est analysé au moyen d'un système d'essai spécifique au marqueur sur des marqueurs de maladies pulmonaires.

11. Procédé de fractionnement du volume d'expiration selon l'une quelconque des revendications 8 à 10, **caractérisé en ce que** :
les flux volumiques partiels séparés sont affectés à des zones différentes des voies respiratoires.

12. Utilisation d'un dispositif selon l'une quelconque des revendications 1 à 7 pour le fractionnement du volume d'expiration afin de localiser des modifications dans les voies respiratoires, dans laquelle les volumes partiels séparés du volume d'expiration sont affectés à différentes zones des voies respiratoires.
